# EUROPEAN PATENT SPECIFICATION

(11) **EP 2 373 780 B1**
(45) Date of publication and mention of the grant of the patent: **27.12.2017**
(21) Application number: 09793624.9
(22) Date of filing: 27.11.2009
(51) Int. Cl.: C12M 1/12, C12M 3/04, C12M 1/00, C12M 1/42

(54) **BI-DIRECTIONAL CONTINUOUS PERFUSION BIOREACTOR FOR TRIDIMENSIONAL CULTURE OP MAMMAL TISSUE SUBSTITUTES**
BIDIREKTIONALER KONTINUIERLICHER PERFUSIONSBIOREAKTOR FÜR DREIDIMENSIONALE KULTUR AUS SÄUGERGEWEBESUBSTITUTEN
BIORÉACTEUR À PERFUSION EN CONTINU BIDIRECTIONNELLE POUR CULTURE EN TROIS DIMENSIONS DE SUBSTITUTS TISSULAIRES MAMMIFÈRES

(30) Priority: 04.12.2008 PT 08104278
(43) Date of publication of application: 12.10.2011
(73) Proprietor: Association For The Advancement Of Tissue Engineering And Cell Based Technologies & Therapies (A4TEC), 4710-057 Braga (PT)
(72) Inventor: GARDEL, Leandro, 4400-345 Vila Nova de Gaia (PT); GOMES,Maria Manuela Estima, 4250-543 Porto (PT); REIS, Rui Luís Gonçalves dos, 4250-242 Porto (PT)
(74) Representative: Magalhães Simões, José Raúl
(86) International application number: PCT/PT2009/000063
(87) International publication number: WO 2010/064943

(56) References cited:
- WO-A2-2006/041893
- DE-A1-102005 009 065
- US-A- 4 988 623
- US-A- 5 712 154
- US-A1- 20010 041 363
- US-A1- 20050 002 910
- US-A1- 20060 223 175
- DATABASE WPI Week 200146 Thomson Scientific, London, GB; AN 2001-426154 XP002580647 & CN 1 290 742 A (INST. MECHANICS CHINESE ACAD. SCI.) 11 April 2001 (2001-04-11)

## Description

### FIELD OF THE INVENTION

The present invention relates to a bi-directional continuous perfusion bioreactor that supports three-dimensional growth of mammal tissue in hybrid materials (cell-material).

### BACKGROUND OF THE INVENTION

Tissue engineering and regenerative medicine are multidisciplinary scientific fields that combine knowledge of material sciences, and biosciences and that provide the opportunity of contributing to the regeneration of injured human tissues due to an illness or accident.

In this sense, the main objective of these areas consists in the development of biological substitutes, which are obtained by seeding a particular type of cell in a biodegradable and biocompatible polymer support, which is then allowed to grow in very specific conditions within an advanced culture system, until a functional hybrid material is obtained, capable of replacing the original tissue.

The support material will function as a substrate for cell adhesion and as a temporary template for new tissue growth, while it is degraded in the body. Despite the significant advances produced by this research field in the recent years, there are still many aspects that limit the achievement of such an ambitious objective.

One of the limitations is related to the culture systems currently available, which do not allow to culturing large size hybrid materials (cell - material) and therefore do not allow obtaining functional tissue substitutes for the regeneration of large tissue defects.

Currently the majority of culture systems (see U.S. Patent 4 649 117, U.S. 6 943 008, U.S. 4 649 118, U.S. 5 989 913, U.S. 5 763 279, U.S. 4 537 860, U.S. 6 218 182, U.S. 6 943 008; U.S. 6 991 933, U.S. 6 875 605, U.S. 5 712 154, U.S. 6 607 910, U.S. 5 026 650, U.S. 4 988 623, U.S. 4 184 916) are based on two types of culture system: static and flow perfusion.

Most of the culturing systems, besides the standard static plates or flasks, involve medium agitation (provided by magnetic bar, for example) around the hybrid material. On the other hand, the perfusion flow systems are mostly based on the use of peristaltic pumps to create a directional control of the culture medium within the culture chambers containing the cell-material constructs.

Both the static type system and the flow perfusion currently commercialized, only allow for an enhanced exchange of nutrients and the removal of metabolic products, around the three-dimensional (3D) support (scaffold), as compared to the culture in the standards flasks/well-plates. Due to this limitation it is only possible to grow small three-dimensional (3D) supports, hence creating a limitation of clinical use, indispensable for the future development of this scientific field.

In particular, WO 2006/041893 A2 discloses an apparatus for seeding material in a porous scaffold member capable of entrapping seeding material therein, comprising: a chamber having an interior and capable of maintaining a negative pressure and capable of enclosing a scaffold member therein; and a support member for rotating the scaffold member disposed within the interior of the chamber and for introducing the seeding material into the chamber.

The present invention has the advantage of providing engines with the ability to promote ability to movement at 360°/360° and 180°/180° with a maximum of 52 RPM, which has the effect of supporting the growth of large functional 3D tissues.

US 4988623 discloses a bio-reactor system including an outer first tubular member having closed ends for defining an enclosed cell culture vessel, said tubular member having a horizontally extending, longitude, central axis; first means for rotating said first tubular member about said central axis; a central second tubular member mounted within said first tubular member for rotation about said central axis, said second tubular member and said first tubular member defining an annular cell culture chamber; second means for rotating said second tubular member about said central axis; an intermediate blade means mounted in said annular cell culture chamber for rotation about said central axis; third means for rotating said blade means about said central axis; where said first, second and third means for rotating are capable of providing independent rotation with respect to one another; and first passage means for connecting an input fluid to said cell culture chamber and second passage means for connecting said cell culture chamber and said second tubular member for output of fluid from said cell culture chamber whereby fluid can be passed through said cell culture chamber while said tubular members and blade means are being rotated.

The ability to grow 3D tissues is mainly associated with the microgravity used in this document, unlike the present invention, where low rotation speed plays a role in such process.

Other documents and references are herein mentioned as further relevant background art:
US 2006 0223 175 A1 discloses a bioreactor system for cultivating tissue cells comprising a vessel containing a gas phase and a liquid phase; at least one substrate on which the tissue cells are attached; and a movable shaft to which the substrate is fixed, the movable shaft carrying the substrate into and out of the gas and liquid phases so as to apply shear stress to the tissue cells.

DATABASE WPI Week 200146, Thomson Scientific, London, GB; AN 2001-426154 and CN 1290742 A, disclosing a cultivator having three concentric sleeves constituting the inner chamber for culture solution to flow, the middle cell culturing chamber and the outer air chamber. In the walls of the inner sleeve and the middle sleeve there are liquid to liquid exchange film and air to liquid exchange film separately. The three sleeves are rotated in the same or different angular speed. A liquid pipe to a liquid pump is connected to the pipe inside the inner sleeve, and the air pipe to an air pump is connected to the inlet port of the air chamber. During the culture, the culture body and the culture liquid are filled into the cultivator and the shearing stress is controlled from zero to the physiological stress of cell or tissue.

US 20010041363 A1 discloses a bioartificial device, comprising a semipermeable surface coated with a layer of non-immortalized endothelial cells, epithelial cells, or a mixture thereof and pericyte, vascular smooth muscle or mesangial cells or a mixture thereof, wherein one side of said semipermeable surface is in fluid communication with a blood inlet and the other side is in fluid communication with a blood outlet.

US5712154 A discloses a cell culture unit comprising a shell having two ends and defining an elongated chamber there between; a liquid and a gas perfusion port located on each of said end; liquid perfusion fibers connected to said liquid perfusion ports and gas perfusion fibers connected to said gas perfusion ports; said fibers defining extracapillary and intracapillary space; means communicating with the intracapillary space of said gas perfusion fibers; means communicating with the intracapillary space of said liquid perfusion fibers; and means communicating with said extracapillary space.

DE102005009065 A discloses a method for the preservation of defined cell clusters comprising the steps of: (1) providing a carrier matrix suitable for colonization with biological cells, (2) colonizing the carrier matrix with biological cells, and (3) defined freezing of the colonized carrier matrix (18) along a temperature gradient. The following step (2a) is carried out after step (2): (2a) rinsing the carrier matrix with the addition of a protective substance. The freezing is carried out by introducing the settled carrier matrix (18) into liquid nitrogen, in such a way that the carrier matrix (18) is cooled from a temperature of 0 ° C to 45 ° C over a period of 0,1 to 2 hours, preferably of about 50 minutes ; C, preferably 10 ° C, to a temperature of -40 ° C to -200 ° C, preferably to -100 ° C.

US20050002910 A discloses a method of growing new bone or bone-like tissue under *in vitro* cell culture conditions comprising providing ground demineralized bone and bone-forming cells in a bioreactor under conditions sufficient to form bone or bone-like biomaterial suitable for transplantation by causing a flow of nutrient solutions into, through, and out of the bioreactor.

### BRIEF DESCRIPTION OF THE INVENTION

The bi-directional continuous perfusion bioreactor (BCPB) as defined in the claims will solve this technical problem presented by these bioreactors, through an innovative design which consists in the use of a rigid cylindrical tube and with perforations in the central part with equal length to the three-dimensional support (of large dimensions) that will be used (variable), which connected to a circular system will have the function of promoting the flow passage from the interior to the exterior of the three-dimensional support (linked to the peristaltic pump) or from exterior to the interior of the three-dimensional support(connection to the vacuum pump). This tube will be positioned coaxially within a cell culture chamber with four exits/entrances and hermetically closed, connected to the same circular system, which will allow the control of the flow and of the pressure gradient inside the chamber and in the perforated tube.

The BCPB was designed with the main objective of solving the limitation of the currently commercially available bioreactors, which do not allow growing large-size three-dimensional (3D) supports to be used in tissue engineering and regenerative medicine applications.

Therefore, the BCPB creates the possibility to control the flow and pressure gradient either inside or outside the large-size three-dimensional support. Its main advantage is related precisely with the possibility of controlling the culture medium from within the cell culture chamber and vice versa, thereby allowing a more efficient access to nutrients to cells within the interior of the material and removal of metabolic products from the interior of the material.

### DESCRIPTION OF THE DRAWINGS

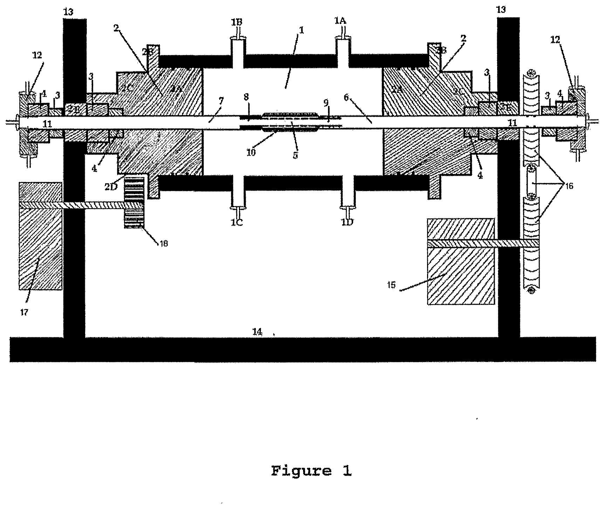

Figure 1 represents the bioreactor object of this invention. In order to facilitate the understanding of its components, these are identified according to the list below:
**Reference 1:** Cell Culture Chamber
   1A; 1B; 1C; 1D : Exits/entrances of the cell culture chamber
**Reference 2:** Cylindrical lids of the cell culture chamber, which are composed of five distinct components (References 2A-2E).
   **Reference 2 A:** Part of the lid that is in direct contact with the cell culture chamber and which prevents draining of the culture medium (with the help of an o-ring)
   **Reference 2 B:** Backstop of the part 2A
   **Reference 2 C:** Part that contains the rolling systems and the retainers
   **Reference 2 D:** Part where is fixed the gear-wheel
   **Reference 2 E**: Part of the support system of the cell culture chamber
**Reference 3:** Rolling system (stainless steel)
**Reference 4:** Silica retainer that prevents medium drainage
**Reference 5:** Coaxial perforated tube
**Reference 6** and **7:** Parts that connect (and fixate) the coaxial perforated tube to the main tubes (that provide the connexion to the exterior tubes of the system)
**Reference 8:** Mechanism of connection that allows the sliding of the perforated coaxial tube.
**Reference 9:** Mechanism of connection (screws) of the perforated coaxial tube.
**Reference 10:** Scaffold.
**Reference 11:** Main tube that connects the perforated tube to the exterior.
**Reference 12:** Three way stopcock.
**Reference 13:** Part of the support systems that connects the cell culture chamber (through part 2E) to the support base.
**Reference 14:** Support base.
**Reference 15:** Rotational engine 1.
**Reference 16:** System of the pulley connected to engine 1.
**Reference 17:** Rotational engine 2.
**Reference 18:** System of gear-wheel connected to engine 2.

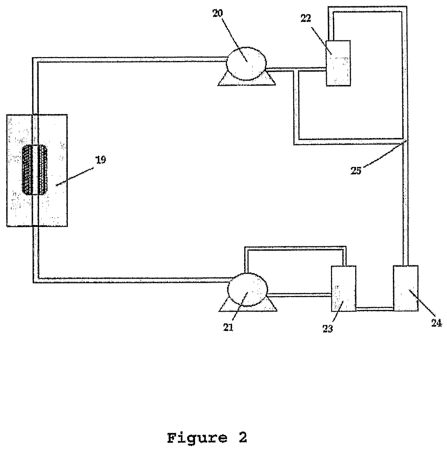

Figure 2 shows the pneumatic project of the circular flow system with:
   **Reference 19:** Cell Culture chamber.
   **Reference 20:** Peristaltic pump.
   **Reference 21:** Bottle reservoir of culture medium.
   **Reference 22:** Collecting bottle of culture medium.
   **Reference 23:.Three** way stopcock for control of flow direction.

In figure 2 it is represented the whole culture system, in which this invention bioreactor is inserted. Its components are identified according to the list below:
**Reference 19:** Cell Culture chamber.
**Reference 20:** Peristaltic pump.
**Reference 21:** Bottle reservoir of culture medium.
**Reference 22:** Collecting bottle of culture medium.
**Reference 23:** Three way stopcock for control of flow direction.

### DETAILED DESCRIPTION OF THE INVENTION

The main distinction between the BCPB and other existing and commercially available bioreactors is the possibility of controlling the culture medium flow from within the three-dimensional support to the interior of the cell culture chamber and vice versa, thereby allowing the nutrients access to the material interior and the removal of metabolic products from the material interior.

The BCPB is directly related to cell culture and will improve particularly the methods and cell culture devices in three-dimensional support currently available in the market, being constituted by a cell culture chamber (1) composed of a cylindrical transparent part, made of acrylic or polycarbonate, with four output/input possibilities (1A, 1B, 1C, 1D), which can be used for: connection to three-way valves that will be connected to silicone tubes where culture medium will flow; to sensors for measuring O₂, CO₂, pH, temperature, pressure gauges etc.; door access to enable sampling or for the inoculation of cells or other substances, such as antibiotics or growth factors.

In each of the two free ends of the cell culture chamber, two parts will be embedded (2), similar to each other and made of Teflon®: one of the pieces made up of four separate parts and the other composed of five distinct parts. The first part (2a) is cylindrical and is responsible for the contact with the inner part of the cell culture chamber, promoting a hermetic and waterproof sealing through the use of two sealing rings. The function of the second part (2B) is to stop the first one. It presents a hexagon placed in level with the other end of the piece, allowing the positioning of the entire bioreactor when placed on a flat surface. The third part (2c) has a cylindrical shape and presents a difference in one of the two pieces that will be part of a cogwheel (2D). Both parts contain a compartment which is placed in a docking system of waterproof rolling stainless steel (3) and silicone retainer (4) to allow a waterproof seal of the cell culture chamber, providing a rotational movement of 360 degrees to the coaxial perforated tube (5) . The fifth part of this piece (2E) will be cylindrical allowing the support of the entire system to the arms (13) that connect to the support base (14), which will keep the whole system sustained when in operation. It will also have the function to support the 180-180 degrees motion to which the cell culture chamber will be submitted.

In order to promote the connection of the perforated coaxial tube to the bearing system, two stainless steel or polycarbonate tube pieces will be used (6.7), which will go through the entire length of the piece that contains the bearing system, extending itself beyond its outer limit (11). Meanwhile, each of these pieces will have a different connection mechanism to the perforated coaxial tube, being one of the connections made by screws (9) and the other by a plug in sliding mechanism (8).

In the perforated coaxial tube (5), one of the ends will present a thread and the other one a smooth and rounded part. The length and the number of holes will be determined by the size of the three-dimensional support (10). This also determines the length that should be slid through the perforated coaxial tube in the interior of the connection piece to the bearing system so that a perfect sealing is established. The stainless steel or polycarbonate tubes coming from within the bioreactor (11) will be connected to the continuous circular flow system through a three-way valve (12) on both sides. Their rotational movement should be neutralized through the use of another bearing system (3, 4), so that a self convolution around the axis of every pipes connected does not occur.

Two engines will be coupled to the BCPB: the first one will be responsible for the 360 degrees rotational movement, with a maximum of 52 RPM, of the perforated coaxial tube. This driving force will be transmitted to the tube through a pulley system (16). The second will be responsible for the 180-180 and 360 degrees rotational movement, with a maximum of 52 RPM of the cell culture chamber (17), this driving force will be transferred to the cell culture chamber by a gear system (18).

For the operation of the BCPB we designed a new circular flow system that is composed of a peristaltic pump (20) responsible for the positive flow gradient in the cell culture chamber and perforated coaxial tube, a vacuum pump (21) responsible for the negative flow gradient in the cell culture chamber and the perforated coaxial tube, a reservoir bottle of the peristaltic pump (22), a reservoir bottle of the vacuum pump (23), and a collector bottle of the culture medium (24). A three-way valve is positioned between the collector bottle and the reservoir bottle of the peristaltic pump, which will function, when desired, for directing the flow directly to the peristaltic pump, thus allowing the exchange of a new culture medium with the old culture medium in the reservoir bottle of the peristaltic pump, without being necessary to stop the entire flow system.

### EMBODIMENTS OF THE INVENTION

a) Bi-directional continuous flow bioreactor characterized in that it comprises the following elements:
   - cell culture chamber (1) with four possibilities of input/output (1A, 1B, 1C, 1D);
   - two connection pieces (2), consisting of a (2A) rounded, smooth part responsible for the contact with the inner part of the cell culture chamber promoting the hermetic and waterproof sealing through the use of two sealing rings, a second part (2B) which serves to backstop for the first part, presenting a hexagonal shape, a third party that will present a smooth rounded shape in one of its pieces (2C) and a cog in the other one (2D) which will fit another cogwheel;
   - stainless steel coaxial tube (5), round, smooth, containing a series of holes made in its entire outer diameter, where the size and total number of holes is determined by the size of the three-dimensional support (scaffold) (10) to be used;
   - two stainless steel tubes (6, 7), round, smooth, that serve as a connection to the perforated coaxial tube (5), using a sliding system (8) for connection to the coaxial tube perforated in one of the tubes (7) and a screw system (9) for connection to the perforated coaxial tube (5) in the other tube (6);
   - two connection pieces (2) that present a rolling system containing a silicone retainer (4) in the interior of the third section (2C) that seals the interior of the cell culture chamber and a waterproof stainless steel rolling system(3);
   - a roller that connects to a pulley system (16) attached on one side of the stainless steel tubes (6, 7) ;
   - connection of the stainless steel tubes (6, 7), in the exterior of the system, connected to a continuous circular flow system through a three-way stopcock (12) on both sides, in which their rotational movements are neutralized by using a bearing system (3, 4) to prevent the winding around their axis of all pipes connected to them;
   - an engine connected to a driving axle by a cogwheel system which will connect to cogwheel (2D) of the bioreactor promoting movement throughout the system and another engine connected to a driving axle by a sheave in which a pulley system will be connected (2D) which will connect the other coupled sheave in one of the sides of the connection tube (11) promoting movement to the perforated coaxial tube, wherein characterized in that the engines can promote movement at 360°/360° and 180°/180° degrees with a maximum of 52 RPM; and by
   - a circular flow system that is associated with the continuous bi-directional flow reactor characterized by being constituted by the following components:
      - a peristaltic pump (20) responsible for the positive flow gradient in the cell culture chamber and in the perforated coaxial tube;
      - a vacuum pump (21) responsible for the negative gradient flow in the cell culture chamber and in the perforated coaxial pipe;
      - a reservoir bottle of the peristaltic pump (22);
      - a reservoir bottle of the vacuum pump (23);
      - a collector bottle for the culture medium (24);
      - a three-way stopcock (25) positioned between the collector bottle and the reservoir bottle of the peristaltic pump, which will function, when desired, by directing the flow of the collector bottle directly to the peristaltic pump.
b) Bio-reactor according to the above mentioned, characterized in that the cell culture chamber (1) is constituted of rounded and transparent acrylic with two openings at the ends, and four inputs/outputs.
c) Bioreactor, according to the above mentioned, characterized in that the input/output (1A, 1B, 1C, 1D) is constituted by an acrylic pipe connected to the interior of the acrylic tube of the cell culture chamber, through a perforation created on it through a screw system, which will be connected to a male/female connecting piece with a three-way valve to which silicone tubes will be connected, where the culture mediums will flow; O2, CO2, pH, temperature, pressure gauges and other measurement sensors; access doors for sampling or for cells inoculation, or inoculation of substances such as antibiotics, growth factors, among others.
d) Bioreactor according to the previous claims, characterized in that both of the stainless steel tubes (6, 7) passing through within the bearing system (3, 4), can promote a 360 degrees rotational movement in the perforated coaxial tube (5).
e) Continuous bi-directional flow bioreactor, according to the previous claims, characterized in that it induces mechanical cellular stimuli through the creation of shear forces caused by the flow perfusion using different pressure gradients.

## Claims

1. Bi-directional continuous flow bioreactor **characterized in that** it comprises the following elements:
- cell culture chamber (1) with four possibilities of input/output (1A, 1B, 1C, 1D);
- two connection pieces (2), consisting of a (2A) rounded, smooth part responsible for the contact with the inner part of the cell culture chamber promoting the hermetic and waterproof sealing through the use of two sealing rings, a second part (2B) which serves to backstop for the first part, presenting a hexagonal shape, a third party that will present a smooth rounded shape in one of its pieces (2C) and a cog in the other one (2D) which will fit another cogwheel;
- stainless steel coaxial tube (5), round, smooth, containing a series of holes made in its entire outer diameter, where the size and total number of holes is determined by the size of the three-dimensional support (scaffold) (10) to be used;
- two stainless steel tubes (6, 7), round, smooth, that serve as a connection to the perforated coaxial tube (5), using a sliding system (8) for connection to the coaxial tube perforated in one of the tubes (7) and a screw system (9) for connection to the perforated coaxial tube (5) in the other tube (6);
- two connection pieces (2) that present a rolling system containing a silicone retainer (4) in the interior of the third section (2C) that seals the interior of the cell culture chamber and a waterproof stainless steel rolling system(3);
- a roller that connects to a pulley system (16) attached on one side of the stainless steel tubes (6, 7) ;
- connection of the stainless steel tubes (6, 7), in the exterior of the system, connected to a continuous circular flow system through a three-way stopcock (12) on both sides, in which their rotational movements are neutralized by using a bearing system (3, 4) to prevent the winding around their axis of all pipes connected to them;
- an engine connected to a driving axle by a cogwheel system which will connect to cogwheel (2D) of the bioreactor promoting movement throughout the system and another engine connected to a driving axle by a sheave in which a pulley system will be connected (2D) which will connect the other coupled sheave in one of the sides of the connection tube (11) promoting movement to the perforated coaxial tube, **characterized in that** the engines can promote movement at 360°/360° and 180°/180° degrees with a maximum of 52 RPM; and by
- a circular flow system that is associated with the continuous bi-directional flow reactor being constituted by the following components:
- a peristaltic pump (20) responsible for the positive flow gradient in the cell culture chamber and in the perforated coaxial tube;
- a vacuum pump (21) responsible for the negative gradient flow in the cell culture chamber and in the perforated coaxial pipe;
- a reservoir bottle of the peristaltic pump (22);
- a reservoir bottle of the vacuum pump (23);
- a collector bottle for the culture medium (24);
- a three-way stopcock (25) positioned between the collector bottle and the reservoir bottle of the peristaltic pump, which will function, when desired, by directing the flow of the collector bottle directly to the peristaltic pump.

2. Bio-reactor according to claim 1, **characterized in that** the cell culture chamber (1) is constituted of rounded and transparent acrylic with two openings at the ends, and four inputs/outputs.

3. Bioreactor, according to the previous claims, **characterized in that** the input/output (1A, 1B, 1C, 1D) is constituted by an acrylic pipe connected to the interior of the acrylic tube of the cell culture chamber, through a perforation created on it through a screw system, which will be connected to a male/female connecting piece with a three-way valve to which silicone tubes will be connected, where the culture mediums will flow; O2, CO2, pH, temperature, pressure gauges and other measurement sensors; access doors for sampling or for cells inoculation, or inoculation of substances such as antibiotics, growth factors, among others.

4. Bioreactor according to the previous claims, **characterized in that** both of the stainless steel tubes (6, 7) passing through within the bearing system (3, 4), can promote a 360 degrees rotational movement in the perforated coaxial tube (5)

5. Continuous bi-directional flow bioreactor, according to the previous claims, **characterized in that** it induces mechanical cellular stimuli through the creation of shear forces caused by the flow perfusion using different pressure gradients.

## Patentansprüche

1. Bidirektionaler Bioreaktor mit kontinuierlichem Fluss, **gekennzeichnet dadurch, dass** er die folgenden Bestandteile enthält:
- Zellkulturkammer (1) mit vier Eingangs- und Ausgangsmöglichkeiten (1A, 1B, 1C, 1D);
- zwei Verbindungsstücke (2), bestehend aus einem (2A) abgerundeten, glatten Teil, das für den Kontakt mit dem inneren Teil der Zellkulturkammer zuständig ist und die hermetische und wasserdichte Abdichtung mit Hilfe von zwei Dichtungsringen fördert, einem zweiten Teil (2B), das als Fänger für den ersten Teil dient und eine hexagonale Form aufweist, und einem dritten Teil, dass einerseits teilweise eine glatte, abgerundete Form aufweist (2C) und andererseits teilweise zahnförmig ist (2D), so dass in ein anderes Zahnrad passt;
- ein koaxiales Edelstahlrohr (5), rund, glatt, mit einer Reihe von Löchern über den gesamten äußeren Durchmesser hinweg, wobei die Größe und Gesamtanzahl der Löcher von der Größe der dreidimensionalen Stütze (Gerüst) (10) abhängt, die verwendet wird;
- zwei Edelstahlrohre (6, 7), rund, glatt, die als Verbindung mit dem durchlöcherten koaxialen Rohr dienen und ein Schiebesystem (8) für die Verbindung zum koaxialen Rohr mit einem der Rohre (7) verwenden, und ein Schraubsystem (9) zur Verbindung des durchlöcherten koaxialen Rohrs (5) mit dem anderen Rohr (6);
- zwei Verbindungsstücke (2), die ein rollendes System mit einem Silikonhalter (4) im inneren des dritten Abschnitts (2C) darstellen, das das Innere der Zellstrukturkammer und ein wasserdichtes Rollsystem aus Edelstahl (3) abdichtet;
- eine Rolle, die sich mit einem Seilzugsystem (16) verbindet, das an einer Seite der Edelstahlrohre (6, 7) angebracht ist;
- eine Verbindung der Edelstahlrohre (6, 7) außen am System), die mit einem andauerndem Kreislaufsystem durch einen Dreiwege-Absperrhahn (12) an beiden Seiten verbunden ist, in dem die kreisenden Bewegungen durch ein Lagersystem (3, 4) neutralisiert werden, um das Drehen aller verbundenen Rohre um ihre eigene Achse zu verhindern;
- ein Motor, der über ein Zahnradsystem mit einer Antriebswelle verbunden ist, das mit dem Zahnrad (2D) des Bioreaktors verbunden ist und für die Bewegung durch das System sorgt, und ein weiterer Motor, der über eine Umlenkrolle mit einer Antriebswelle verbunden ist, mit der ein Seilzugsystem (2D) verbunden wird, das die andere verbundene Umlenkrolle mit einer der Seiten des Verbindungsrohrs (11) verbindet und die Bewegung des durchlöcherten Edelstahlrohrs bewirkt, **gekennzeichnet dadurch, dass** die Motoren eine Bewegung von 360°/360° und 180°/180° bei einer maximalen Umdrehungszahl von 52 UpM ermöglichen; und durch
- ein Kreislaufsystem, das mit dem bidirektionaler Reaktor mit kontinuierlichem Fluss verbunden ist, der aus den folgenden Komponenten besteht:
- einer Schlauchpumpe (20), die für den positiven Flussgradienten in der Zellkulturkammer und dem durchlöcherten Edelstahlrohr zuständig ist;
- einer Vakuumpumpe (21), die für den negativen Flussgradienten in der Zellkulturkammer und dem durchlöcherten Edelstahlrohr zuständig ist;
- einer Auffangflasche für die Schlauchpumpe (22);
- einer Auffangflasche für die Vakuumpumpe (23);
- einer Sammelflasche für das Kulturmedium (23);
- einem Dreiwege-Absperrhahn (25), der sich zwischen der Sammelflasche und der Auffangflasche für die Schlauchpumpe befindet, und, wenn gewünscht, den Fluss der Sammelflasche direkt an die Schlauchpumpe leitet.

2. Bioreaktor gemäß Anspruch 1, **gekennzeichnet dadurch, dass** die Zellkulturkammer (1) aus abgerundetem und transparentem Acrylglas mit zwei Öffnungen an den Enden und vier Eingängen/Ausgängen besteht.

3. Bioreaktor gemäß den vorangegangenen Ansprüchen, **gekennzeichnet dadurch, dass** der Eingang/Ausgang (1A, 1B, 1C, 1D) aus einem Acrylrohr besteht, das mit dem inneren des Acrylschlauchs der Zellkulturkammer verbunden ist, und zwar durch eine Perforation, die durch ein Schraubsystem erzeugt wird, das mit einem männlichen/weiblichen Verbinder mit einem Dreiwegeventil verbunden wird, an das Silikonschläuche angeschlossen werden, durch die das Kulturmedium fließt; O2-, CO2-, pH-, Temperatur- und Druckventile sowie weitere Messsensoren; Zugangsklappen zum Entnehmen von Proben oder für die Inokulation von Zellen oder die Inokulation von Stoffen wie Antibiotika, Wachstumsfaktoren usw.

4. Bioreaktor gemäß den vorangegangenen Ansprüchen, **gekennzeichnet dadurch, dass** beide Edelstahlrohre (6, 7), die durch das Lagersystem (3, 4) geführt werden, eine Drehbewegung um 360 Grad im durchlöcherten koaxialen Rohr (5) durchführen können.

5. Bidirektionaler Bioreaktor mit kontinuierlichem Fluss gemäß den vorangegangenen Ansprüchen, **gekennzeichnet dadurch, dass** er mechanische Zellstimulationen durch die Erzeugung von Scherkräften auslöst, die durch die Flussperfusion mit Hilfe von verschiedenen Druckgradienten erzeugt werden.

## Revendications

1. Bioréacteur à flux continu bidirectionnel **caractérisé en ce qu'**il comprend les éléments suivants :
- chambre de culture cellulaire (1) avec quatre possibilités d'entrée/sortie (1A, 1B, 1C, 1D) ;
- deux pièces de raccordement (2), consistant en une (2A) partie arrondie, lisse responsable du contact avec la partie interne de la chambre de culture cellulaire promouvant le scellement hermétique et imperméable par le biais de l'utilisation de deux anneaux d'étanchéité, une deuxième partie (2B) qui sert de butée de la première partie, présentant une forme hexagonale, une troisième partie qui présentera une forme arrondie lisse dans l'une de ses pièces (2C) et une roue dentée dans l'autre (2D) qui s'introduira dans une autre roue dentée ;
- un tube coaxial en acier inoxydable (5), rond, lisse, contenant une série de trous faits dans tout son diamètre externe, où la taille et le nombre total de trous sont déterminés par la taille du support à trois dimensions (échafaudage) (10) à utiliser ;
- deux tubes en acier inoxydable (6, 7), arrondis, lisses, qui servent de raccordement au tube coaxial perforé (5), en utilisant un système de coulissement (8) pour le raccordement au tube coaxial perforé dans l'un des tubes (7) et un système à vis (9) pour le raccordement au tube coaxial perforé (5) dans l'autre tube (6) ;
- deux pièces de raccordement (2) qui présentent un système de roulement contenant un dispositif de retenue en silicone (4) à l'intérieur de la troisième section (2C) qui scelle l'intérieur de la chambre de culture cellulaire et un système de roulement en acier inoxydable imperméable (3) ;
- un galet qui est raccordé à un système de poulie (16) relié à un côté des tubes en acier inoxydable (6, 7) ;
- raccordement des tubes en acier inoxydable (6, 7), à l'intérieur du système, raccordés à un système de flux circulaire continu par le biais d'un robinet d'arrêt à trois voies (12) sur les deux côtés, dans lequel leurs mouvements rotatifs sont neutralisés en utilisant un système de palier (3, 4) pour empêcher l'enroulement autour de leur axe de tous les tuyaux raccordés à ces derniers ;
- un moteur raccordé à un essieu d'entraînement par un système de roue dentée qui se raccordera à la roue dentée (2D) du bioréacteur promouvant le mouvement à travers le système et un autre moteur raccordé à un essieu d'entraînement par une réa dans laquelle un système de poulie sera raccordé (2D) qui raccordera l'autre réa couplée dans l'un des côtés du tube de raccordement (11) promouvant le mouvement au tube coaxial perforé, **caractérisé en ce que** les moteurs peuvent promouvoir le mouvement à 360°/360° et 180°/180° degrés avec un maximum de 52 tr/min ; et par
- un système de flux circulaire qui est associé au réacteur à flux bidirectionnel continu étant constitué des composants suivants :
- une pompe péristaltique (20) responsable du gradient de flux positif dans la chambre de culture cellulaire et dans le tube coaxial perforé ;
- une pompe à vide (21) responsable du gradient de flux négatif dans la chambre de culture cellulaire et dans le tuyau coaxial perforé ;
- une bouteille réservoir de la pompe péristaltique (22) ;
- une bouteille réservoir de la pompe à vide (23) ;
- une bouteille collectrice pour le milieu de culture (24) ;
- un robinet d'arrêt à trois voies (25) positionné entre la bouteille collectrice et la bouteille réservoir de la pompe péristaltique, qui fonctionnera, lorsqu'on le désire, en dirigeant le flux de la bouteille collectrice directement à la pompe péristaltique.

2. Bioréacteur selon la revendication 1, **caractérisé en ce que** la chambre de culture cellulaire (1) est constituée d'acrylique arrondi et transparent avec deux ouvertures aux extrémités, et quatre entrées/sorties.

3. Bioréacteur, selon les revendications précédentes, **caractérisé en ce que** l'entrée/sortie (1A, 1B, 1C, 1D) est constitué d'un tuyau acrylique raccordé à l'intérieur du tube acrylique de la chambre de culture cellulaire, par le biais d'une perforation créée sur celui-ci par le biais d'un système à vis, qui sera raccordé à une pièce de raccordement mâle/femelle avec une vanne à trois voies à laquelle des tubes en silicone seront raccordés, où les milieux de culture circuleront ; O2, CO2, pH, température, manomètres et autres capteurs de mesure ; portes d'accès pour l'échantillonnage ou pour l'inoculation de cellules, ou l'inoculation de substances telles que les antibiotiques, les facteurs de croissance, entre autres.

4. Bioréacteur selon les revendications précédentes, **caractérisé en ce que** les deux tubes en acier inoxydable (6, 7) passant par l'intérieur du système de palier (3, 4), peut promouvoir un mouvement rotatif à 360 degrés dans le tube coaxial perforé (5)

5. Bioréacteur à flux bidirectionnel continu, selon les revendications précédentes, **caractérisé en ce que** cela induit des stimulus cellulaires mécaniques par le biais de la création de forces de cisaillement causées par la perfusion de flux en utilisant différents gradients de pression.
